# EUROPÄISCHE PATENTANMELDUNG

(11) **EP 2 597 139 A1**
(43) Veröffentlichungstag der Anmeldung: **29.05.2013**
(21) Anmeldenummer: 11190450.4
(22) Anmeldetag: 24.11.2011
(51) Int. Cl.: C10L 1/02, C11C 3/00, C07C 67/03

(54) **Oxidationsstabilisierter Biodiesel**

(71) Anmelder: LANXESS Deutschland GmbH, 51369 Leverkusen (DE)
(72) Erfinder: Ingendoh, Axel, Dr., 51519 Odenthal (DE); Böger, Uwe, Dr., 51375 Leverkusen (DE)

(57) **Zusammenfassung**

Die vorliegende Erfindung betrifft oxidationsstabilisierten Biodiesel, ein Verfahren zur Herstellung und dessen Verwendung.

## Beschreibung

Die vorliegende Erfindung betrifft oxidationsstabilisierten Biodiesel, ein Verfahren zur Herstellung und dessen Verwendung.

Biodiesel oder Fettsäurealkylester, insbesondere Fettsäuremethylester oder Fettsäureethylester, wird in zunehmenden Maße als eine Alternative zum herkömmlichen Dieselkraftstoff eingesetzt. Er hat sich als regenerativer und CO₂ neutraler Kraftstoff der ersten Generation etabliert.

Die Herstellung erfolgt fast ausschließlich durch Umesterung von Pflanzenölen (Fettsäureglyceriden), wie beispielsweise Palmölen, Rapsöl, Sojaöl oder Sonnenblumenöl, mit Methanol oder Ethanol zu Fettsäuremethyl- bzw. -ethylestem durch Katalyse von Basen, wie NaOH, KOH, Natrium- oder Kaliummethylat.

Pflanzenöle sind im Gegensatz zu Mineralölen stärkeren Alterungsprozessen unterlegen. Die erhöhte Oxidation sowie die Bildung freier Fettsäuren sind im Wesentlichen für diese Alterung verantwortlich. Daher hat sich der Einsatz von Antioxidantien mehr und mehr etabliert. So ist beispielsweise aus EP-A 1736528 der Einsatz von substiutierten Alkylphenolen bekannt. Diese werden in Konzentrationen 0,005 bis 2,0 Gew.% dem Biodiesel zugefügt.

Als Zusatz ist in diesem Zusammenhang vor allem Butylhydroxytoluol (BHT) bekannt. Dieses wird ebenfalls in Mengen von 0,005 bis 2,0 Gew.-% dem Biodiesel zugefügt.

Es ist jedoch wünschenswert, die Oxidationsstabilität des Biodiesels noch weiter zu erhöhen.

Aufgabe der vorliegenden Erfindung war es daher, einen verbesserten oxidationsstabilen Biodiesel bereitzustellen, der den Herstellern ein breiteres Zeitfenster bei der Verarbeitung und Lagerung ermöglicht.

Überraschenderweise wurde nun festgestellt, dass die Oxidationsstabilität des Biodiesels dadurch deutlich erhöht werden kann, dass bestimmte Antioxidatien bereits dem natürlichen Öl vor der Umesterung zugesetzt werden. Dies ermöglicht sogar, aus altem natürlichen Öl noch Biodiesel herzustellen, was ohne diese Behandlung nicht möglich wäre.

Gegenstand der vorliegenden Erfindung ist daher ein oxidationsstabilisierter Biodiesel (Fettsäurealkylester), erhältlich durch die Dotierung mindestens eines natürlichen Öls mit mindestens einer Verbindung der Formel (I) 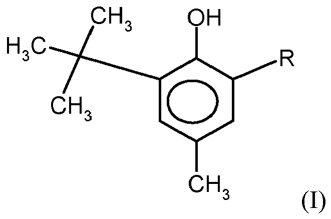 mit 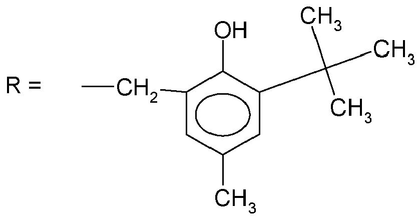 oder R = tert.-Butyl
und anschließender Umesterung des natürlichen Öls in Gegenwart von Methanol und/oder Ethanol.

Dabei ist als Verbindung der Formel (I) Butylhydroxtoluol (BHT) 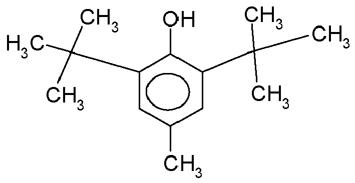 bevorzugt.

In einer anderen Ausführungsform der Erfindung ist als Verbindung der Formel (I) 2,2'-Methylenbis(6-tert-butyl-4-methylphenol), d.h. 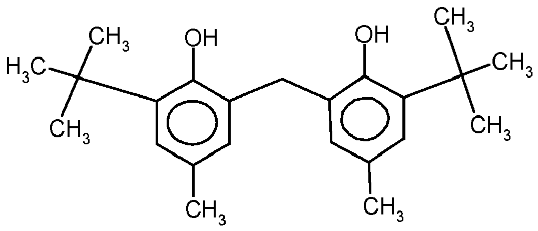 bevorzugt.

Bei den Verbindungen der Formel (I) handelt es sich um handelsübliche Verbindungen, die z.B. bei der Lanxess Deutschland GmbH unter dem Handelsnamen Baynox® (Vulkanox® BHT) oder Baynox® plus (Vulkanox® BKF) erhältlich sind.

Unter natürlichen Ölen werden dabei pflanzliche Öle, wie vorzugsweise Rapsöl, Sojaöl, Palmöle oder andere Pflanzenöle, aber auch Altspeiseöle, z. B. aus der Nahrungsmittelherstellung, und Algenöl und tierische Fette verstanden. Chemisch handelt es sich bei diesen natürlichen Ölen um Fettsäureglyceride. Bevorzugt werden Pflanzenöl eingesetzt. Besonders bevorzugt sind dabei pflanzliche Öle, wie Rapsöl oder Sojaöl.

Unter Dotierung im Sinne der Erfindung wird vorzugsweise das Einbringen von mindestens einer Verbindung der Formel (I) in einem natürlichen Öl verstanden. Das Einbringen erfolgt dabei vorzugsweise durch Mischen bis zum Auflösen der Verbindung der Formel (I).

Die Dotierung des natürlichen Öls mit mindestens einer Verbindung der Formel (I) erfolgt dabei vorzugsweise durch Mischen, vorzugsweise Einrühren von mindestens einer Verbindung der Formel(I) in kristalliner Form und/oder durch das Einrühren einer konzentrierten Lösung von 10 bis 20 Gew% der Verbindung der Formel (I) in natürlichem Öl und/oder einer konzentrierten Lösung von 20 bis 50 Gew% der Verbindung der Formel (I) gelöst in Biodiesel in das natürliche Öl bei einer Temperatur von vorzugsweise 40 bis 50°C.

Als Mischaggregate sind prinzipiell alle bekannten Rührorgane, vorzugsweise axiale, radiale oder tangentiale Rührorgane, wie beschrieben in Wilke et al., Rührtechnik Verfahrenstechnische und apparative Grundlagen. Dr. Alfred Hüthig Verlag Heidelberg, 2.Auflage, 1991, Seiten 92 bis 97, geeignet..

Die Mengen an Verbindung der Formel (I) betragen vorzugsweise 0,005 bis 2,0 Gew.%.

Bei Biodiesel im Sinne der Erfindung handelt es sich um Fettsäuremethylester (FAME) und/oder ein Fettsäureethylester (FAEE), d. h., dass das bei der Umesterung eingesetzte Alkanol entweder Methanol oder Ethanol ist. Auch Mischungen aus FAME oder FAEE sind möglich, d. h., dass in diesem Fall ein Alkanolgemisch aus Methanol und Ethanol eingesetzt wird.

Die Umesterung wird dabei vorzugsweise alkalisch durchgeführt. In dieser Ausführungsform der Erfindung wird vorzugsweise das natürliche Öl, vorzugsweise Rapsöl oder Sojaöl, und das Alkanol, vorzugsweise Methanol oder Ethanol, sowie der Katalysator vorzugsweise NaOH, KOH, Natriummethanolat NaOCH₃ oder andere Methanolate in Methanol gelöst, vorgelegt, und auf Temperaturen im Bereich von vorzugsweise 50 bis 80 Grad Celsius erwärmt.

Das Mengenverhältnis von natürlichem Öl zu Alkanol beträgt vorzugsweise 1200g zu 260g. Die Reaktionslösung wird dann vorzugsweise bei Temperaturen von 50 bis 80°C für eine Zeitdauer von ca. 80 bis 180 Minuten gerührt. Als Mischaggregate sind dabei alle bekanten Rührorgane geeignet. Anschließend werden die Phasen der Reaktionslösung getrennt und die Biodieselphase mit Wasser gewaschen. Aus der Biodieselphase wird dann das Restwasser, z. B. durch Destillation (Anstrippen) entfernt. Üblicherweise erhält man mit dem erfindungsgemäßen Verfahren Biodiesel mit einer Ausbeute von über 90%.

In dieser Ausführungsform der Erfindung wird der Katalysator, vorzugsweise NaOH, KOH, Natriummethanolat NaOCH₃ oder andere Methanolate, in Methanol gelöst, zum Methanol gegeben und aufgelöst.

Die Menge an Katalysator beträgt vorzugsweise 0,5-1,5 Gew%. Das Methanol wird über das stöchiometrische Verhältnis von Öl (Glycerinester) zu Alkohol hinaus zugegeben, um die Reaktion auf die Seite des Methylesters zu verschieben.

Nach der Beendigung der Reaktion liegt das Gemisch in zwei Phasen vor. Die leichtere Phase enthält Biodiesel mit Beimengungen von Methanol, die untere Phase hauptsächlich Glycerin und Nebenprodukte. Nach Abtrennung der Unterphase wird der Biodiesel zweimal mit 10 bis 30 Gew.% Wasser gewaschen um Spuren von Lauge sowie das Methanol zu entfernen und entweder destilliert oder anderweitig getrocknet.

Die Glycerinphase enthält überschüssiges Methanol sowie die meisten Nebenprodukte des Prozesses.

Gegenstand der Erfindung ist zudem ein Verfahren zur Herstellung des erfindungsgemäßen Biodiesels, wonach, dass mindestens ein natürliches Öls mit mindestens 0,005 bis 2,0 Gew.% einer Verbindung der Formel (I) 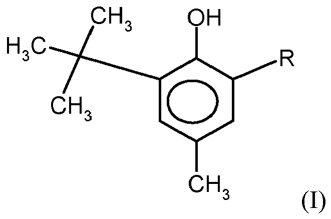 mit 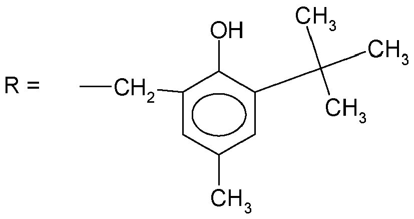 oder R = tert.-Butyl
dotiert und
dieses anschließend in Gegenwart von Methanol und/oder Ethanol umgeestert wird.

In Bezug auf die Verfahrensbedingungen und auf die eingesetzten Mengen wird auf die obigen Ausführungen verwiesen.

Gegenstand der vorliegenden Erfindung ist zudem die Verwendung des erfindungsgemäß stabilisiertem Biodiesel als Kraftstoff.

Die folgenden Beispielen sollen die Erfindung erläutern, ohne sie jedoch in ihrem Umfang zu beschränken.

### Beispiele

Durchführung der Rancimat-Tests:
Für den Rancimat-Test wurde der Rancimat 679, ein Gerät der Firma Metrohm AG, eingesetzt. Dieser bestand aus einem Steuerteil und einem Nassteil. Im Nassteil wurden die Proben erhitzt und in Gegenwart von Kupfer von Luft durchblasen. Während der oxidativen Alterung entstanden niederkettige, flüchtige organische Säuren, die in eine mit destilliertem Wasser gefüllte Messzelle eingeleitet wurden. Dort wurde die Leitfähigkeit kontinuierlich erfasst und aufgezeichnet.

Das Ende der Alterung bzw. oxidativen Stabilität zeigte sich durch einen steilen Anstieg der Leitfähigkeit an. Die Zeit bis zum Erreichen des Knickpunktes wird als Induktionsperiode bezeichnet und dient als Maß für die Alterungsstabilität.

In den folgenden Beispielen wurden alle Proben der gleichen Versuchsanforderung unterworfen:
Dauer: 120 Minuten bei 70°C unter Durchleitung von 60 ml/h Luft/Std. Als Antioxidant wurde vor allem Butylhydroxytoluol (BHT) der Firma Lanxess Deutschland eingesetzt, erhältlich unter dem Handelsnamen Baynox®.

### Beispiel 1: Lagerstabilität von Rapsöl/Sojaöl nach Behandlung mit Baynox® zum Vergleich

Eingesetzt wurde Rapsöl bzw. Sojaöl, in das kein, bzw. 500 ppm bzw. 1000 ppm Baynox® bei 40°C eingerührt wurde.

Die nach dem o.g. Rancimat-Test erhaltene Lagerstabilität ist für Rapsöl aus Tabelle 1 und für Sojaöl aus Tabelle 2 aufgeführt.

**Tabelle 1:**

| **Lagerung** | **V 1** | | **V-2** | | **V-3** | |
|---|---|---|---|---|---|---|
| **40°C** | **Rancimat** | **Baynox®** | **Rancimat** | **Baynox®** | **Rancimat** | **Baynox®** |
| **[Tage]** | **[h]** | **[ppm]** | **[h]** | **[ppm]** | **[h]** | **[ppm]** |
| **Start** | **2,6** | **ohne Baynox®** | **4,4** | **500** | **5,5** | **1000** |
| **7 Tage** | **2,9** | | **4,5** | **451** | **5,7** | **954** |
| **14 Tage** | **2,6** | | **4,4** | **427** | **5,8** | **937** |
| **21 Tage** | **2,3** | | **4,3** | **404** | **5,6** | **904** |
| **28 Tage** | **1,7** | | **3,8** | **383** | **5,0** | **904** |

| | | | | | | |
|---|---|---|---|---|---|---|
| *Bestimmung der Oxidationsstabilität =Rancimat-Methode* | | | | | | |

**Tabelle 2:**

| **Lagerung** | **V4** | | **V5** | | **V6** | |
|---|---|---|---|---|---|---|
| **40°C** | **Rancimat** | **Baynox®** | **Rancimat** | **Baynox®** | **Rancimat** | **Baynox®** |
| **[Tage]** | **[h]** | **[ppm]** | **[h]** | **[ppm]** | **[h]** | **[ppm]** |
| **Start** | **3,2** | **ohne Baynox®** | **4,4** | **500** | **5,2** | **1000** |
| **7 Tage** | **2,8** | | **4,3** | **494** | **5,3** | **977** |
| **14 Tage** | **2,6** | | **4,1** | **477** | **5,0** | **963** |
| **21 Tage** | **2,4** | | **3,9** | **464** | **4,9** | **960** |
| **28 Tage** | **2,0** | | **3,3** | **452** | **4,5** | **945** |

Rapsöl ohne Antioxidant (V1) hat nach 28 Tagen Lagerung bei 40°C die niedrigste Oxidationsstabilität, d.h. Rapsöl ohne Antioxidant alterte deutlich schneller. Alleine durch die Zugabe von 500 ppm Baynox® in das Rapsöl ist die Oxidationsstabilität nach 28 Tagen doppelt so hoch. Der Alterungsprozess wird deutlich abgeschwächt.

Auch Sojaöl ohne Antioxidant (V4) zeigte nach 28 Tagen Lagerung bei 40°C die niedrigste Oxidationsstabilität. Durch die direkte Zugabe von Baynox® in das Sojaöl wurde auch hier der Alterungsprozess des Sojaöls abgeschwächt. Sojaöl mit einer Anfangsdotierung von 500 ppm Baynox® als Antioxidant hat nach einer Lagerzeit von 28 Tagen eine um 65% höhere Oxidationsstabilität als Sojaöl ohne Antioxidant. Eine Anfangsdotierung von 1000 ppm mit Baynox® erhöht die Oxidationsstabilität um 125%.

### Beispiel 2: Oxidationsstabilität von Rapsöl/Sajoöl nach Umesterung und Behandlung mit Baynox ®

Die Proben V1 bis V6 werden mit 146,8 g Methanol bei 65 °C in Gegenwart von 4,09g Natriumhydroxid umgeestert.

Die Proben V1 und V4, die bislang kein Baynox® enthielten wurden umgeestert und der Rancimattest nach Zugabe von 0 bzw. 500 ppm Baynox® durchgeführt.

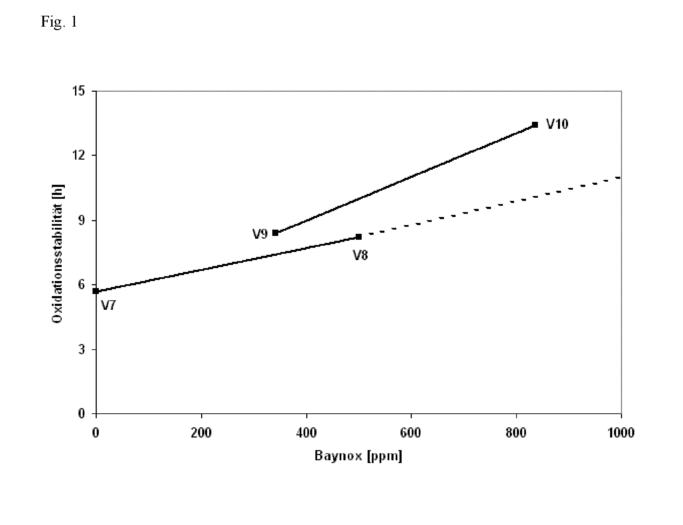

Die Ergebnisse sind in den Figuren 1 (Rapsöl) und 2 (Sojaöl) dargestellt:
Dabei werden folgende Kürzel benutzt:
   V7 = Biodiesel nach Umesterung von V1 ohne Baynox®,
   V8 = Biodiesel nach Umesterung von V 1 und anschließender Zugabe von 500 ppm Baynox®,
   V9= Biodiesel nach Umesterung von V2,
   V10 = Biodiesel nach Umesterung von V3,
   V11 = Biodiesel nach Umesterung von V4 ohne Baynox®,
   V12 = Biodiesel nach Umesterung von V4 und anschließender Zugabe von 500 ppm Baynox®,
   V 13= Biodiesel nach Umesterung von V5 und
   V14 = Biodiesel nach Umesterung von V6.

Dabei zeigte sich, dass der Zeitpunkt der Zugabe von Baynox® entscheidend ist für die Oxidationsstabilität des Biodiesel.

Überraschenderweise wurde gefunden, dass Biodiesel aus natürlichen Ölen, dem Baynox®, direkt vor der Umesterung in das Pflanzenöl zugesetzt wird, eine deutlich bessere Qualität hat (siehe Figur1) als Biodiesel, dem erst nach der Umesterung das Antioxidanz zugesetzt wurde.

V7 und V8 zeigten die Oxidationsstabilität des Biodiesels, der aus dem undotierten Rapsöl hergestellt wurde. Dieser wies eine Anfangsoxidationsstabilität von 5,7 Stunden auf. Nach Zugabe von 500 ppm Baynox® erhöhte sich die Oxidationsstabilität auf 8,2 Stunden. V9 und V10 zeigten die Oxidationsstabilitäten der Biodiesel, welcher aus den mit anfangs 500 ppm bzw. 1000 ppm dotierten Rapsöl hergestellt wurde. V9 zeigte mit einem Restgehalt von 341 ppm Baynox® eine höhere Oxidationsstabilität als V8 mit 500 ppm Baynox®. Noch deutlicher ist dieser Effekt bei V10 zu erkennen, der aus dem mit 1000 ppm Baynox® dotierten Rapsöl hergestellt wurde.

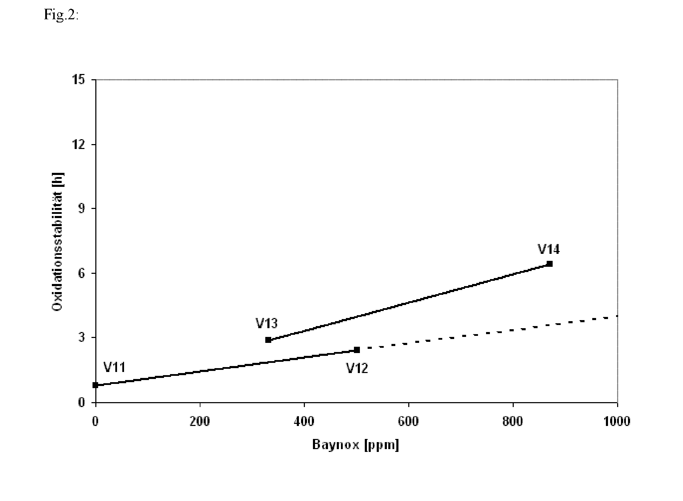

Das gleiche Ergebnis zeigt sich auch bei Versuchen zur Lagerstabilität von Sojaöl anstelle von Rapsöl (siehe Figur 2).

V11 zeigte die Oxidationsstabilität des Biodiesels, der aus dem undotiertem Sojaöl hergestellt wurde und eine Anfangsoxidationsstabilität von 0,8 Stunden aufwies. Nach Zugabe von 500 ppm Baynox® erhöhte sich die Oxidationsstabilität auf 2,4 Stunden. V11 erreichte selbst mit 1000 ppm Baynox® nicht die Europäische Biodieselnorm von 6 Stunden Oxidationsstabilität.

Für V13 und V14 wurden die Oxidationsstabilitäten des Biodiesels gemessen, die aus den mit anfangs 500 ppm bzw. 1000 ppm dotiertem Sojaöl hergestellt wurden.

V13 zeigt mit einem Gehalt von 332 ppm Baynox® eine höhere Oxidationsstabilität als der V12 nach Dotierung mit 500 ppm Baynox®. Auch hier ist der Effekt wieder deutlich bei dem Biodiesel V14 zu erkennen, der aus dem mit 1000 ppm Baynox® dotierten Sojaöl hergestellt wurde.

### Beispiel 3: (Vergleich) Lagerstabilität von Rapsöl ohne Baynox®

Rapsöl wurde offen bei Raumtemperatur über 33 Wochen gelagert.

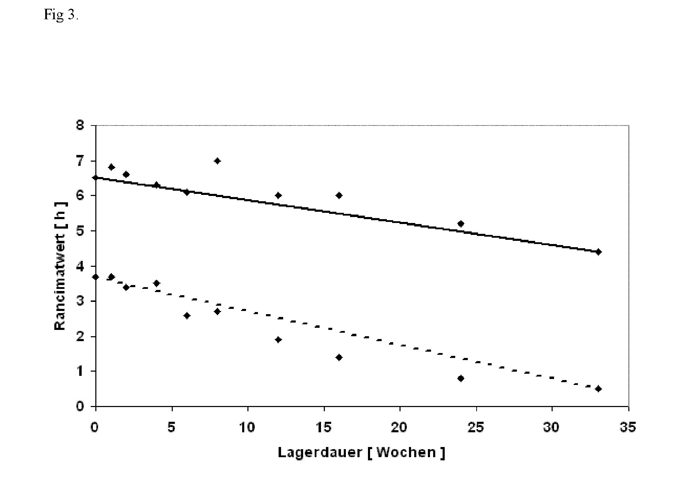

Die Ergebnisse der durchgeführten Rancimatbestimmung sind in Figur 3 (gestrichelte Linie) wiedergegeben.

Nach nur wenigen Monaten ist Rapsöl mit einer Anfangs-Oxidationsstabilität von 3,7 Stunden auf 0,5 Stunden abgefallen.

### Beispiel 4: (Vergleich) Lagerstabilität von Rapsöl mit Baynox®

Rapsöl wurde mit 1000 ppm Baynox® versetzt und offen bei Raumtemperatur über 33 Wochen gelagert.

Die Ergebnisse der durchgeführten Rancimatbestimmung sind in Figur 3 (durchgezogene Linie) wiedergegeben. Rapsöl mit einem Anfangsgehalt von 1000 ppm Baynox® verfügt nach 33 Wochen Lagerzeit immer noch über eine Oxidationsstabilität von 4,4 Stunden.

### Beispiel 5: (Vergleich) Herstellung von Biodiesel aus gealtertem Rapsöl ohne Baynox®

Rapsöl wurde offen bei Raumtemperatur über 33 Wochen gelagert (analog zu Beispiel 3). Daraus mittels alkalischer Umesterung hergestellter Biodiesel erreichte nur eine Oxidationsstabilität von 0,6 Stunden. Auch mit Zusatz großer Mengen Antioxidant (bis 2500 ppm Baynox®) lässt sich aus diesem Rapsöl kein spezifikationsgerechter Biodiesel mehr herstellen.

### Beispiel 6: (erfindungsgemäß) Herstellung von Biodiesel aus gealtertem Baynox®-haltigem Rapsöl

Stabilisiertes und gelagertes Rapsöl mit 1000 ppm Baynox® aus Beispiel 4 wies nach alkalischer Umesterung einen Biodiesel mit einem Baynox®-Gehalt von 740 ppm Baynox® und einer Oxidationsstabilität von 7,3 Stunden auf. Der Alterungsprozess war abgeschwächt und die Haltbarkeit des Öls deutlich erhöht.

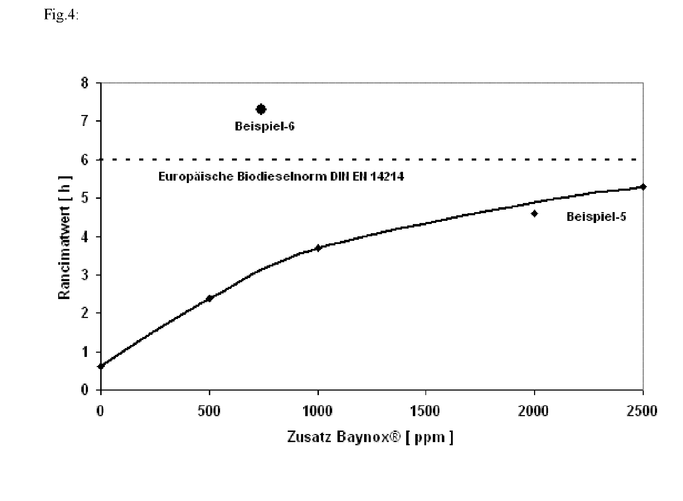

Die Ergebnisse der Beispiele 5 und 6 sind in der Figur 4 dargstellt.

Die Ergebnisse des Vergleichsbeispiels sind mit einer durchgezogenen Linie, die Biodieselnorm bei 6 Stunden mit einer gestrichelten Linie und der Messwert für das erfindungsgemäße Beispiel 6 als Einzelwert dargestellt.

### Durch die direkte Zugabe von Baynox® in das Pflanzenöl vor der Umesterung ist es möglich auch nach langer Lagerzeit aus dem Öl noch spezifikationsgerechten Biodiesel herzustellen.

### Beispiel 7:

Der Versuch mit Baynox®-haltigem Rapsöl und direkter Umesterung zu Biodiesel zeigte eine höhere Oxidationsstabilität (Versuch 1044-B = 11,5 Stunden, Versuch 1052 = 9,5 Stunden) als bei Biodiesel aus Baynox®-freiem Rapsöl und nachträglicher Zugabe von 500 ppm Baynox®. (Versuch 1044-A = 6,4 Stunden).

| **Vers.Nr.** | | **Baynox®** | | **Baynox®** | | |
|---|---|---|---|---|---|---|
| | **Pflanzen** | **Zusatz** | **Analyse** | **Zusatz** | **Analyse** | **Rancimat** |
| **XBOU-** | **ÖL** | **[ppm]** | **[ppm]** | **[ppm]** | **[ppm]** | **[h]** |
| **1044-A** | **Raps-Öl** | **kein Zusatz** | | **500** | **439** | **6,4** |
| **1044-B** | **Raps-Öl** | **500** | **446** | **kein Zusatz** | **423** | **11,5** |
| **1052** | **Raps-Öl** | **500** | **509** | **kein Zusatz** | **503** | **9,5** |

Bei den analog zu BHT mit 2,2'-Methylenebis(6-tert-butyl-4-methylphenol) (Vulkanox® BKF) durchgeführten Versuchen wurde die positive Effekt der Zudosierung vor der Umesterung auf die Oxidationsstabilität des Biodiesels bestätigt.

## Patentansprüche

1. Stabilisierter Biodiesel (Fettsäurealkylester), erhältlich durch die Dotierung mindestens eines
natürlichen Öls mit mindestens einer Verbindung der Formel (I) 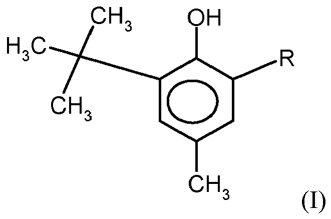 mit 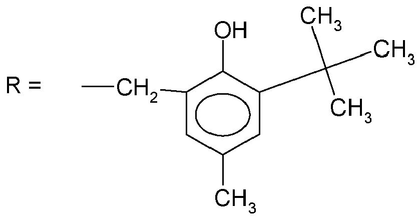 odertert.-Butyl,
und anschließender Umesterung. des natürlichen Öls in Gegenwart von Methanol und/oder Ethanol.

2. Biodiesel nach Anspruch 1, **dadurch gekennzeichnet, dass** das natürliche Öl ein Pflanzenöl ist.

3. Biodiesel nach Anspruch 1 oder 2, **dadurch gekennzeichnet, dass** das natürliche Öl Sojaöl oder Rapsöl ist.

4. Biodiesel nach einem oder mehreren der Ansprüche 1 bis 3, **dadurch gekennzeichnet, dass** es
sich bei der Verbindung der Formel (I) um 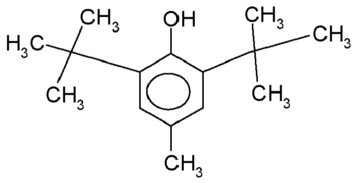 handelt.

5. Biodiesel nach einem oder mehreren der Ansprüche 1 bis 4, **dadurch gekennzeichnet, dass** mindestens ein natürliches Öls mit mindestens 0,005 bis 2,0 Gew% einer Verbindung der Formel (I) 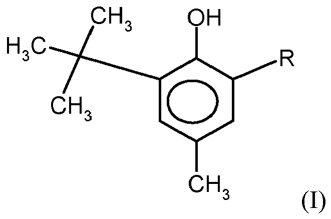 mit 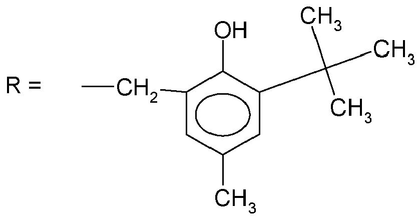 oder tert.-Butyl dotiert und dieses anschließend in Gegenwart von Methanol und/oder Ethanol umgeestert wird.

6. Verfahren zu Herstellung von stabilisiertem Biodiesel nach einem oder mehreren der Ansprüche 1 bis 5, **dadurch gekennzeichnet, dass** die Umesterung des natürlichen Öls in einem Temperaturbereich von 50 bis 80 Grad Celsius erfolgt.

7. Verwendung von stabilisiertem Biodiesel nach einem oder mehreren der Ansprüche 1 bis 5 als Kraftstoff.
